# EUROPEAN PATENT APPLICATION

(11) **EP 4 803 017 A1**
(43) Date of publication of application: **09.09.2026**
(21) Application number: 24885582.7
(22) Date of filing: 24.10.2024
(51) Int. Cl.: A61B 17/12

(54) **SUTURING MEMBER**

(30) Priority: 31.10.2023 JP 2023186995
(71) Applicant: Zeon Corporation, Tokyo 100-8246 (JP); National University Corporation Kagawa University, Takamatsu-shi, Kagawa 760-8521 (JP)
(72) Inventor: SUGITANI, Tatsuro, Tokyo 100-8246 (JP); NISHIYAMA, Noriko, Kita-gun, Kagawa 761-0793 (JP); KOBARA, Hideki, Kita-gun, Kagawa 761-0793 (JP)
(74) Representative: Maiwald GmbH
(86) International application number: PCT/JP2024/037897
(87) International publication number: WO 2025/094809

(57) **Abstract**

Provided is a suturing member that enables hemostasis in a mucosal defect and promotes healing of the mucosal defect. The suturing member 100A used to suture a mucosal defect in a digestive tract wall comprises an annular wire 110 branching into two wire portions at an annular wire base end 120 and is connected at an annular wire tip end 130 opposing the annular wire base end 120, and a tubular member 500 that receives therein the annular wire 110 so as to be slidable from the annular wire base end 120 to the annular wire tip end 130. The annular wire 110 forms a loop portion 200 at a distal side of the tubular member 500, and when the loop portion 200 is positioned so that its approximate center overlaps the approximate center of the mucosal defect, the loop portion 200 has a wire (e.g., a bridging wire 300) that is positioned above the mucosal defect, and the tubular member 500 receives therein the annular wire 110 and the wire (e.g., the bridging wire 300) that is positioned above the mucosal defect.

## Description

### TECHNICAL FIELD

The present invention relates to a suturing member used to suture mucosal defects in the gastrointestinal wall.

### BACKGROUND ART

Conventionally, endoscopic submucosal dissection (ESD) and endoscopic mucosal resection (EMR) are known procedures for resecting lesions in the gastrointestinal wall, such as the large intestine, esophagus, and stomach.

ESD and EMR involve resecting the mucosa where the lesion occurred in the gastrointestinal wall, leaving a mucosal defect, which is a scar (wound) from the resection of the lesion. For example, in ESD, a mucosal defect occurs after resecting the entire lesion, including the submucosa. A mucosal defect caused by ESD is a hole created in a part of the gastrointestinal wall (the site where the lesion was located), with the bottom surface of the mucosal defect reaching the submucosa or near the muscular layer. If bleeding from the mucosal defect is observed, a wound closure procedure is performed to close the opening of the mucosal defect.

One known wound closure procedure is a technique called purse string suture. Purse string suture, for example, uses a detachable snare with a roughly circular loop for ligating internal tissue, as described in Patent literature 1. The detachable snare is positioned to surround the mucosal defect, and multiple points of the detachable snare are secured to the mucosa around the edge of the mucosal defect (peripheral mucosa) with endoscopic clips or the like. The detachable snare is then pulled and squeezed to close the opening of the mucosal defect like the opening of a purse string.

### CITATION LIST

### PATENT LITERATURE

Patent literature 1: Japanese patent application publication No. 2015-192725

### SUMMARY OF THE INVENTION

### PROBLEM TO BE SOLVED BY THE INVENTION

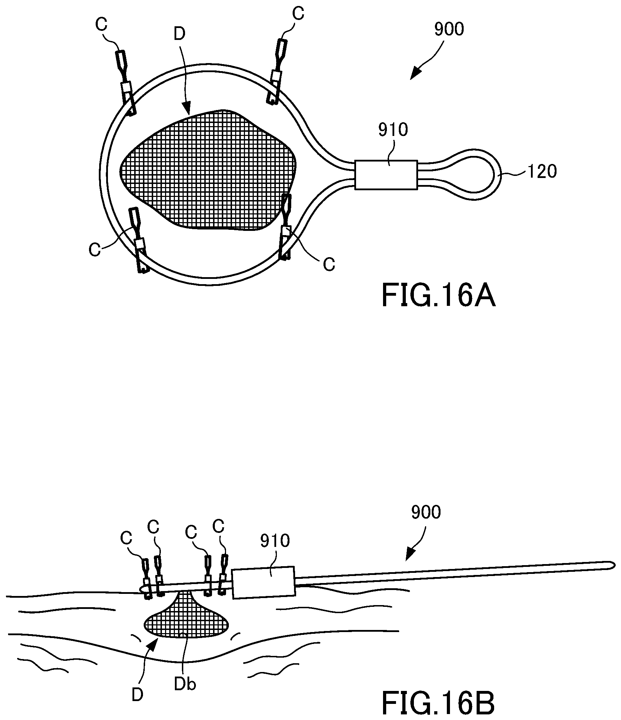

In conventional purse-string sutures, a detachable snare 900 with a substantially circular loop is used, as shown in FIGS. 16A and 16B. First, as shown in FIG. 16A, the detachable snare 900 is secured to the peripheral mucosa with multiple endoscopic clips C. Then, as shown in FIG. 16B, the tubular member 910 is slid to tighten the loop of the detachable snare 900 and reduce its diameter, thereby suturing the mucosal defect D.

However, although the opening of the mucosal defect is closed, a void (pocket) is formed inside the mucosal defect D, as shown in FIG. 16B. In other words, the opening of the mucosal defect is closed like squeezing the opening of a pouch, but a cavity forms at the bottom surface Db of the mucosal defect, like the bag part of a pouch. As a result, it is not possible to apply pressure to the bottom surface Db of the mucosal defect, which is the source of bleeding, to stop bleeding, and there is a problem that bleeding cannot be stopped sufficiently. Furthermore, because the bottom surface Db of the mucosal defect is not in a tightly closed state due to the formation of a cavity, tissue healing is hindered, resulting in a problem of delayed healing of the mucosal defect D.

The present invention is made in consideration of the above problems, and aims to provide a suturing member that can stop bleeding in mucosal defects and promote healing of mucosal defects.

### MEANS FOR SOLVING THE PROBLEMS

To achieve the above objectives, the suturing member according to the present invention is a suturing member used to suture a mucosal defect in a digestive tract wall comprising an annular wire that branches into two wire portions at an annular wire base end and is connected at an annular wire tip end opposing the annular wire base end to form an annular shape, and a tubular member that is slidable from the annular wire base end toward the annular wire tip end, the annular wire forming a loop portion at a distal side of the tubular member, and the suturing member further comprises a wire positioned above the mucosal defect when the approximate center of the loop portion overlaps the approximate center of the mucosal defect, and the tubular member receives therein the annular wire and the wire positioned above the mucosal defect.

With the above configuration, when suturing a mucosal defect, the wire of the suturing member is positioned above the mucosal defect. Therefore, the portion of the annular wire that is positioned above the peripheral mucosa can be secured to the peripheral mucosa with an endoscopic clip or the like, and the portion of the wire that is positioned above the mucosal defect can be inserted into the mucosal defect and secured to the bottom surface of the mucosal defect (submucosal layer or muscular layer) with an endoscopic clip or the like.

When the tubular member is slid toward the annular wire tip end while secured with an endoscopic clip or the like, the diameter of the loop portion contracts, and the secured position converges to approximately the center of the loop portion, closing the opening of the mucosal defect and lifting the bottom surface of the mucosal defect toward the opening of the mucosal defect, thereby reducing the void inside the mucosal defect. This makes it possible to stop bleeding from the bottom surface of the mucosal defect and to promote healing of the mucosal defect.

In the above configuration of the suturing member according to the present invention, the wire positioned above the mucosal defect may is constituted by a bridging wire formed to connect two different points of the annular wire in the loop portion.

With the above configuration, the bridging wire spanning across the inside of the loop portion can be positioned above the mucosal defect.

In the above configuration of the suturing member according to the present invention, one end of the bridging wire may be connected to the annular wire base end, and the other end of the bridging wire may be connected to the annular wire tip end.

With the above configuration, the bridging wire is disposed so that the extension direction of the bridging wire coincides with the sliding direction of the tubular member. This allows the tubular member to slide smoothly toward the annular wire tip end after being secured with an endoscopic clip or the like.

In the above configuration of the suturing member according to the present invention, the bridging wire may be curved.

With the above configuration, the bridging wire can be lengthened to provide sufficient slack, preventing deformation of the loop portion due to tension in the bridging wire and allowing the loop surface of the loop portion to be a flat surface that can be easily positioned near the mucosal defect. Furthermore, lengthening the bridging wire makes it easier to insert the bridging wire into the mucosal defect and to secure the bridging wire to the bottom of the mucosal defect with an endoscopic clip or the like.

In the suturing member according to the present invention, in the above configuration, the length of the bridging wire may be approximately the same as the length of the annular wire from the annular wire base end to the annular wire tip end.

With the above configuration, when the annular wire base end and the annular wire are pulled in opposite directions to form a shape that brings them closer to each other, the bridging wire becomes in a state of not being loosed. Therefore, when the tubular member is slid toward the annular wire tip end after being secured with an endoscopic clip or the like, the annular wire and the bridging wire is prevented from being slack, thereby allowing the tubular member to slide smoothly toward the annular wire tip end.

In the suturing member according to the present invention, in the above configuration, the bridging wire may be included within the loop surface of the loop portion and curved in a substantially S-shape.

With the above configuration, the bridging wire is reliably lengthened to provide sufficient slack, making it easier for the bridging wire to reach the bottom surface of the mucosal defect, facilitating securement of the bridging wire to the bottom surface of the mucosal defect with an endoscopic clip or the like.

In the suturing member according to the present invention, in the above configuration, the bridging wire may be curved so as to protrude from the loop surface of the loop portion.

With the above configuration, the bridging wire is reliably lengthened to provide sufficient slack, making it easier for the bridging wire to reach the bottom surface of the mucosal defect, making it easier to secure the bridging wire to the bottom surface of the mucosal defect with an endoscopic clip or the like. Furthermore, when the suturing member is positioned near the mucosal defect, the bridging wire is disposed to protrude above the mucosal defect, making it easy to secure the bridging wire with an endoscopic clip or the like.

In the suturing member according to the present invention, in the above configuration, the wires constituting the annular wire may be pre-shaped to have, in the loop portion, an intersection portion at which the wires intersect with each other, and the wire positioned above the mucosal defect may be formed from a portion of the wire constituting the annular wire.

With the above configuration, when the approximate center of the loop portion is positioned so as to overlap with the approximate center of the mucosal defect, a portion of the annular wire having an intersection portion can be positioned above the mucosal defect.

In the suturing member according to the present invention, in the above configuration, the annular wire may be pre-shaped to have a concavely curved portion that is curved so as to be recessed inward of the loop portion, and the wire positioned above the mucosal defect may be formed from a portion of the wire constituting the annular wire.

With the above configuration, when the approximate center of the loop portion is positioned so as to overlap the approximate center of the mucosal defect, a concavely curved portion, which is a portion of the annular wire, can be positioned above the mucosal defect.

The suturing member according to the present invention, in the above configuration, may include a connecting loop portion formed in an annular shape at a proximal side of the tubular member.

With the above configuration, when the tubular member is slid toward the annular wire tip end after being secured with an endoscopic clip or the like, the annular connecting loop portion can be grasped by a suturing device, allowing the tubular member to slide smoothly.

In the suturing member according to the present invention, in the above configuration, the connecting loop portion may be formed from the annular wire.

With the above configuration, the connecting loop portion can be formed from a portion of the annular wire, allowing the connecting loop portion to be provided with a simple configuration.

In the suturing member according to the present invention, in the above configuration, the connecting loop portion may be formed from an annular connecting member attached to the annular wire.

The above configuration makes it possible to form a connecting loop portion using an annular connecting member that can be gripped during suturing, without being limited by the structure of the annular wire.

### BRIEF DESCRIPTION OF THE DRAWINGS

[FIG. 1] A perspective view of a suturing member in a first embodiment of the present invention.
[FIG. 2] A plan view showing an example of an expanded state of a loop portion of the suturing member in the first embodiment of the present invention.
[FIG. 3] A plan view showing an example of a contracted state of the loop portion of the suturing member in the first embodiment of the present invention.
[FIG. 4] A side view of a suturing device used when suturing the suturing member in the first embodiment of the present invention.
[FIG. 5A] A plan view showing a state of the suturing member in the first embodiment of the present invention immediately before being connected to the suturing device.
[FIG. 5B] A side view showing a state of the suturing member in the first embodiment of the present invention immediately before being connected to the suturing device.
[FIG. 6A] A plan view showing a state in which the suturing member in the first embodiment of the present invention is connected to the distal end of the suturing device.
[FIG. 6B] A side view showing a state in which the suturing member in the first embodiment of the present invention is connected to the distal end of the suturing device.
[FIG. 7A] A plan view showing a state in which a tip member of the suturing device abuts against the suturing member in the first embodiment of the present invention.
[FIG. 7B] A side view showing a state in which the tip member of the suturing device abuts against the suturing member in the first embodiment of the present invention.
[FIG. 8A] A plan view showing a state in which the diameter of the suturing member in the first embodiment of the present invention is contracted by the suturing device.
[FIG. 8B] A side view showing a state in which the diameter of the suturing member in the first embodiment of the present invention is contracted by the suturing device.
[FIG. 9] A plan view illustrating another example of the operation of contracting the diameter of the suturing member in the first embodiment of the present invention by the suturing device.
[FIG. 10A] A diagram showing a state in which the suturing member in the first embodiment of the present invention is secured near a mucosal defect, as viewed from above the mucosal defect.
[FIG. 10B] A diagram showing a state in which the suturing member in the first embodiment of the present invention is secured near the mucosal defect, as viewed from the side of the mucosal defect.
[FIG. 11A] A diagram showing a state in which the mucosal defect is sutured by contracting the diameter of the suturing member in the first embodiment of the present invention, as viewed from above the mucosal defect.
[FIG. 11B] A diagram showing a state in which the mucosal defect is sutured by contracting the diameter of the suturing member in the first embodiment of the present invention, as viewed from the side of the mucosal defect.
[FIG. 12] A perspective view of a suturing member in a second embodiment of the present invention.
[FIG. 13A] A plan view of the suturing member in the second embodiment of the present invention.
[FIG. 13B] A side view of the suturing member in the second embodiment of the present invention.
[FIG. 14] A perspective view of a suturing member in a third embodiment of the present invention.
[FIG. 15A] A diagram schematically showing the shape of a loop portion of the suturing member included in the present invention, as a first example in which a bridging wire spans across the inside of the loop portion.
[FIG. 15B] A diagram schematically showing the shape of a loop portion of the suturing member included in the present invention, as a second example in which a bridging wire spans across the inside of the loop portion.
[FIG. 15C] A diagram schematically showing the shape of the loop portion of the suturing member included in the present invention, as a third example in which a bridging wire spans across the inside of the loop portion.
[FIG. 15D] A diagram schematically showing the shape of the loop portion of the suturing member included in the present invention, showing a first example in which the wire that makes up the loop portion has an intersection portion.
[FIG. 15E] A diagram schematically showing the shape of the loop portion of the suturing member included in the present invention, showing a second example in which the wire that makes up the loop portion has an intersection portion.
[FIG. 15F] A diagram schematically showing the shape of the loop portion of a suturing member included in the present invention, showing a third example in which the wire that makes up the loop portion has an intersection portion.
[FIG. 15G] A diagram schematically illustrating the shape of the loop portion of a suturing member included in the present invention, showing a first example in which the wire material constituting the loop portion has a concavely curved portion.
[FIG. 15H] A diagram schematically illustrating the shape of the loop portion of a suturing member included in the present invention, showing a second example in which the wire material constituting the loop portion has a concavely curved portion.
[FIG. 16A] A diagram illustrating the problem to be solved by the present invention, showing a view from above of the mucosal defect, with a detachable snare secured near the mucosal defect.
[FIG. 16B] A diagram illustrating the problem to be solved by the present invention, showing a view from the side of the mucosal defect, with a void (pocket) formed inside the sutured mucosal defect.

### EMBODIMENTS OF THE INVENTION

The suturing member according to the present invention will now be described with reference to the drawings. The suturing member according to the present invention is used to suture mucosal defects resulting from, for example, resection of lesions in the walls of the digestive tract, such as the large intestine, esophagus, or stomach. In this specification, the inside of the patient's body is referred to as the distal or tip side, and the side closest to the user is referred to as the proximal or base side, based on the user operating the device (suturing device) used to suture the suturing member inside the body and an endoscope. The drawings referred to in this specification are not necessarily to an accurate scale relative to the actual dimensions, and some parts have been exaggerated or simplified to schematically illustrate the configuration according to the present invention.

### (First embodiment)

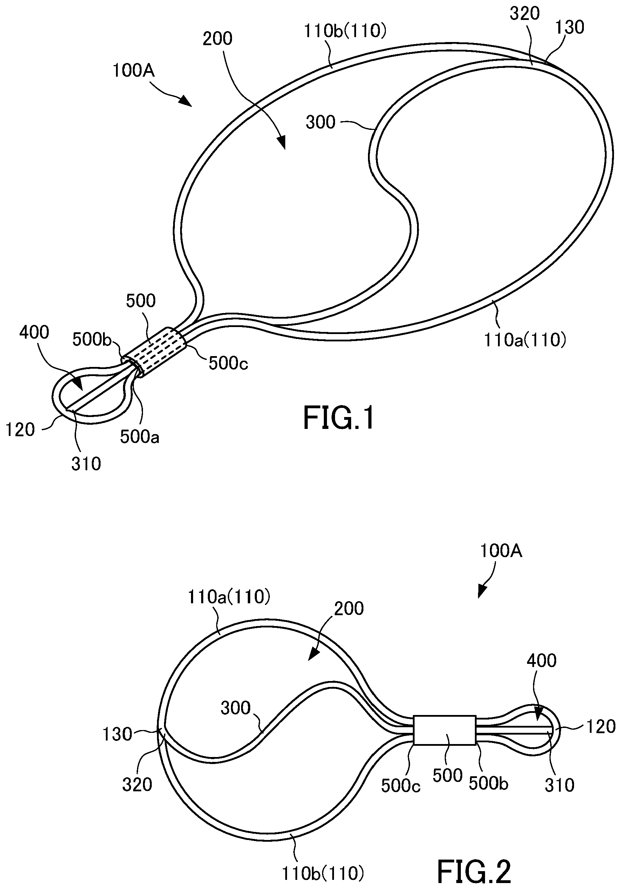

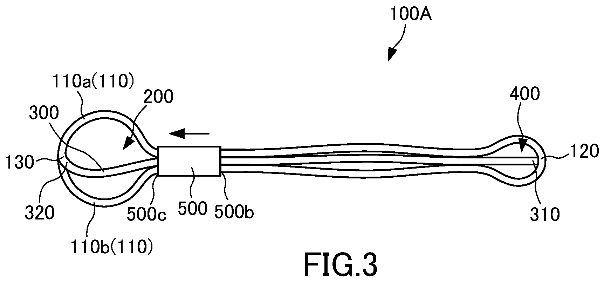

First, the configuration of the suturing member 100A in the first embodiment of the present invention will be described with reference to FIGS. 1 to 3. FIG. 1 is a perspective view showing the suturing member 100A in the first embodiment of the present invention. FIG. 2 is a plan view showing an example of the expanded state of the loop portion 200 of the suturing member 100A in the first embodiment of the present invention. FIG. 3 is a plan view showing an example of the contracted state of the loop portion 200 of the suturing member 100A in the first embodiment of the present invention.

As shown in FIGS. 1 to 3, the suturing member 100A generally comprises an annular wire 110, a bridging wire 300, and a tubular member 500.

The annular wire 110 is formed so that the wires are connected to be formed in an annular shape as a whole. When the proximal (base end) portion of the annular wire 110 is designated as an annular wire base end 120 and the distal (tip end) portion of the annular wire 110 facing the annular wire base end 120 is designated as an annular wire tip end 130, the annular wire 110 is formed so that the annular wire 110 branches into two wire portions at the annular wire base end 120 and is connected at the annular wire tip end 130. In other words, the annular wire 110 has two opposing wires 110a, 110b between the annular wire base end 120 and the annular wire tip end 130. The annular wire 110 can be made of, for example, synthetic polymers such as polyamide, polyester, or polypropylene; natural fibers such as cotton or silk; or metals.

The bridging wire 300 is a wire disposed inside the annular wire 110. In the suturing member 100A in the first embodiment, one end 310 of the bridging wire 300 is connected to the annular wire base end 120, and the other end 320 of the bridging wire 300 is connected to the annular wire tip end 130. In other words, the bridging wire 300 extends to span between the annular wire base end 120 and the annular wire tip end 130. When the bridging wire 300 is disposed so as to connect the annular wire base end 120 and the annular wire tip end 130 in this manner, the extension direction of the bridging wire 300 can be aligned with the sliding direction of the tubular member 500, allowing the tubular member 500 to slide smoothly toward the annular wire tip end 130. The material of the bridging wire 300 is not particularly limited, and the same material as that of the annular wire 110 described above can be used.

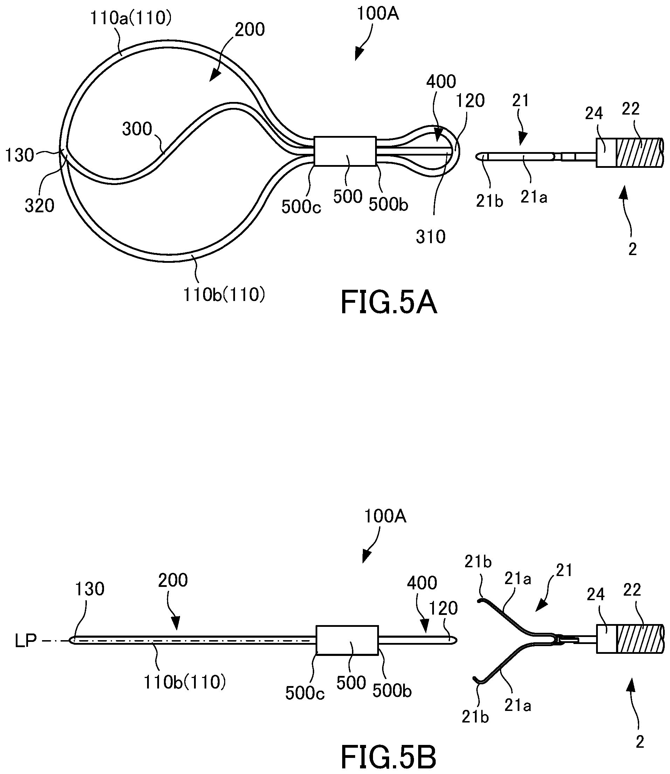

The bridging wire 300 may be formed in a substantially straight line or curved shape between the annular wire base end 120 and the annular wire tip end 130. In the suturing member 100A in the first embodiment, the bridging wire 300 is positioned so as to be contained within the plane formed by the annular wire 110, and is pre-shaped to be curved in a substantially S-shape. By forming the bridging wire 300 in this curved shape, deformation of the loop portion 200 due to the tension of the bridging wire 300 is prevented, and the loop surface LP (see FIG. 5B) of the loop portion 200 can be made into a flat surface that can be easily positioned near the mucosal defect D. Furthermore, increasing the length of the bridging wire 300 makes it easier to insert the bridging wire 300 into the mucosal defect D.

The length of the curved bridging wire 300 is not particularly limited, but in this example, the length of the curved bridging wire 300 is approximately the same as the length of the annular wire 110 from the annular wire base end 120 to the annular wire tip end 130. In other words, the opposing wires 110a, 110b and the bridging wire 300 have approximately the same length. As a result, when the annular wire base end 120 and the annular wire tip end 130 are pulled in opposite directions to form a shape that brings them closer to each other, the bridging wire 300 is in a non-slack state. This prevents slack from occurring in the annular wire 110 and the bridging wire 300 when the tubular member 500 is slid toward the annular wire tip end 130, thereby enabling the tubular member 500 to slide smoothly.

The method of connecting the annular wire 110 and the bridging wire 300 when forming the suturing member 100A is not particularly limited. For example, a single wire may be bent appropriately to form the bridging wire 300, the wire 110a and the wire 110b like a single stroke. Specifically, as shown in FIGS. 1 to 3, the bridging wire 300 may be bent from one end 310 as a starting point to the other end 320 to form the wires 110a, 110b into an annular shape, and then the wires 110a, 110b may be fixed to each other at the annular wire base end 120 and the annular wire tip end 130 by adhesive bonding, fusion bonding, or the like. Alternatively, the annular wire 110 and the bridging wire 300 may be prepared, and the one end 310 and the other end 320 of the bridging wire 300 may be fixed to the annular wire base end 120 and the annular wire tip end 130 by adhesive bonding, fusion bonding, or the like. Furthermore, the annular wire formed by one of the two wires 110a, 110b and the bridging wire 300 may be prepared, and then both ends of the other of the two wires 110a, 110b may be fixed to the annular wire base end 120 and the annular wire tip end 130 by adhesive bonding, fusion, or the like.

The tubular member 500 is a cylindrical member having a lumen 500a that opens at its proximal end face (base end face) 500b and its distal end face (tip end face) 500c. The tubular member 500 receives therein the annular wire 110 so as to be slidable between the annular wire base end 120 and the annular wire tip end 130. At this time, as shown by the dotted lines in FIG. 1, the tubular member 500 has a lumen 500a that is threaded with the two wires 110a, 110b that make up the annular wire 110, as well as the bridging wire 300. The material of the tubular member 500 is not particularly limited, but is preferably elastic, such as fluorine-based resin or silicone rubber.

In the state that the tubular member 500 receives therein the annular wire 110 and the bridging wire 300, an annular loop portion 200 is formed on the distal side (tip side) of the tubular member 500. The loop portion 200 is a ring formed by the annular wire 110 that extends from the vicinity of the distal end face 500c of the tubular member 500 toward the distal side (tip side) of the tubular member 500, so that the loop portion 200 is pre-shaped to form an approximately perfect circle.

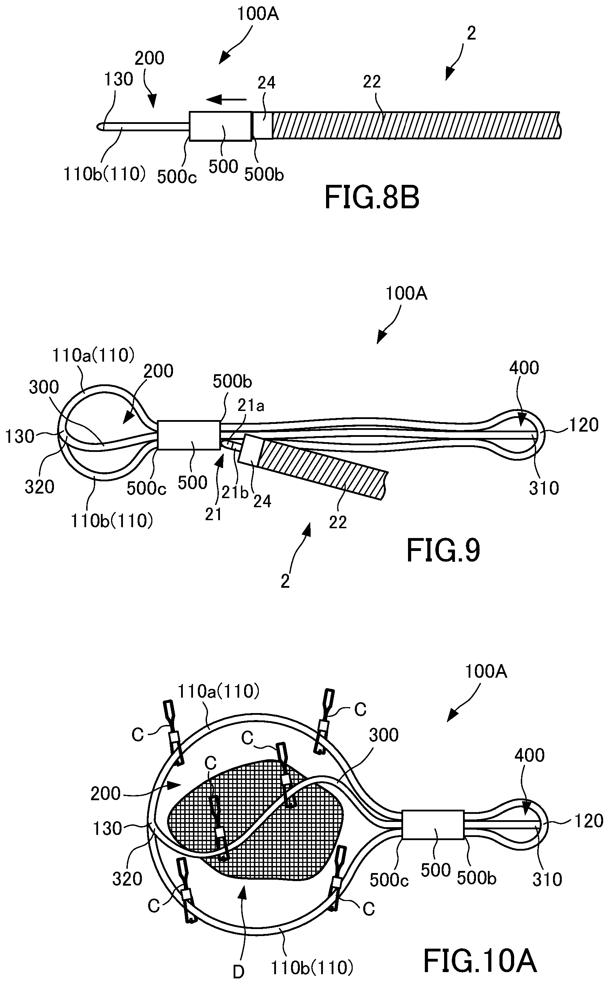

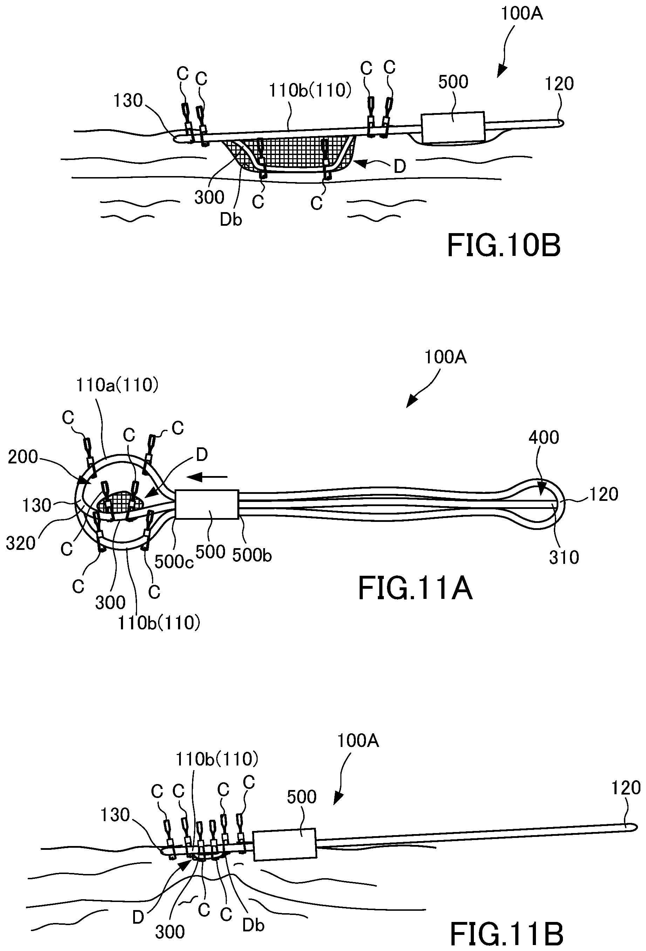

Before suturing the mucosal defect D (see FIG. 10A and FIG. 10B described below), as shown in FIG. 2, the tubular member 500 is positioned on the proximal side (base end side) of the suturing member 100A. At this time, the loop portion 200 formed on the distal side (tip end side) of the tubular member 500 forms a large loop. When the tubular member 500 is slid from the annular wire base end 120 toward the annular wire tip end 130 from this state, the loop portion 200 is squeezed and gradually reduces in diameter as shown in FIG. 3.

Furthermore, in the state that the tubular member 500 receives therein the annular wire 110 and the bridging wire 300, an annular connecting loop portion 400 is formed on the proximal side (base end side) of the tubular member 500. The connecting loop portion 400 is a loop formed by an annular wire 110 extending from the vicinity of the proximal end face 500b of the tubular member 500 toward the proximal side (base end side) of the tubular member 500. As described below, the suturing device 2 grasps the connecting loop portion 400 with its connecting hook 21 and is connected to the suturing member 100A.

The size of the suturing member 100A can be appropriately set according to the size of the mucosal defect D to be sutured. For example, it is preferable to set the diameter of the loop portion 200 larger than the diameter of the opening (mucosal defect opening) of the mucosal defect D to be sutured, so that when the loop portion 200 is positioned so that the approximate center of the mucosal defect D overlaps the approximate center of the loop portion 200, the loop portion 200 will surround the periphery of the opening of the mucosal defect. As an example, the diameter of the loop portion 200 of the suturing member 100A is approximately 2 to 40 mm, and the wire diameters of the annular wire 110 and the bridging wire 300 are approximately 0.2 to 0.6 mm. The tubular member 500 is preferably configured to have the two wires 110a, 110b forming the annular wire 110 and the bridging wire 300 tightly received therein, and the inner diameter of the lumen 500a of the tubular member 500 is approximately 0.5 to 1.5 mm.

As described above, the suturing member 100A in the first embodiment has a configuration in which the tubular member 500 receives therein the annular wire 110 and the bridging wire 300. The loop portion 200 formed on the distal side (tip side) of the tubular member 500 has an annular wire 110 that forms a substantially circular outline in a plan view, and a bridging wire 300 that spans across the inside of the loop portion 200. Sliding the tubular member 500 from the annular wire base end 120 toward the annular wire tip end 130 allows the loop portion 200 to be squeezed and reduced in diameter.

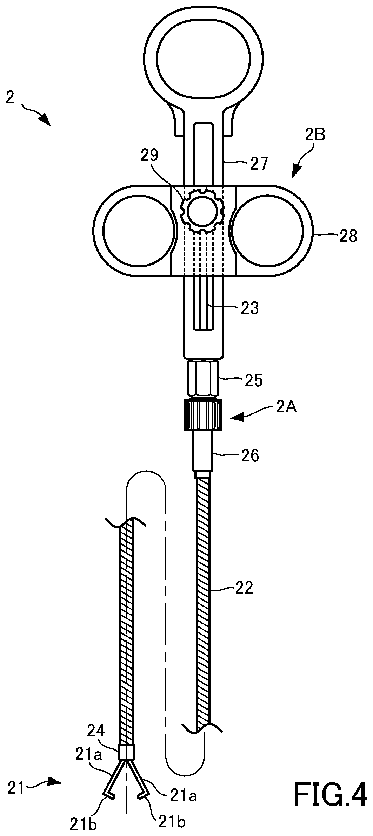

Next, with reference to FIG. 4, the suturing device 2 used with the suturing member 100A in the first embodiment of the present invention will be described. FIG. 4 shows a side view of the suturing device 2 used when suturing the suturing member 100A in the first embodiment of the present invention.

As shown in FIG. 4, the suturing device 2 includes a connecting hook 21, a sheath 22, a drive wire 23, a locking mechanism 2A having a base-side locking member 25 and a sheath-side locking member 26, and an operating unit 2B having a base portion 27 and a slider portion 28.

The sheath 22 is a flexible hollow tube. While a simple tube made of resin or the like may be used as the sheath 22, a coil tube is used in this embodiment. A flat coil tube that is formed by spirally winding a long, flat plate made of a metal such as stainless steel, may be used as the coil tube. However, a round coil tube or an internally flat coil tube may also be used. Furthermore, a wire tube may be used as the sheath 22. A wire tube is a tube made of hollow stranded wires, which are made by twisting a plurality of wires (cables) made of metal such as stainless steel into a spiral shape so as to form a hollow space.

A substantially cylindrical tip member 24, made of, for example, resin, is integrally fixed to the distal end (tip) of the coil tube of the sheath 22. The distal end face of the tip member 24 forms an abutment portion that can abut against the proximal end face (base end face) 500b of the tubular member 500 of the suturing member 100A described above. However, from the perspective of reducing costs and the number of parts, the tip member 24 may not be provided. In that case, the distal end face of the coil tube serves as the abutting portion that abuts against the proximal end face (base end face) 500b of the tubular member 500 of the suturing member 100A.

The drive wire 23 is made of a flexible wire, and in this embodiment, a wire rope is used. The wire rope is a rope made of stranded wires formed by helically twisting multiple wires (cables) made of a metal such as stainless steel. However, a single wire may also be used as the drive wire 23.

A connecting hook 21 is integrally attached to the distal end (tip) of the drive wire 23. The connecting hook 21 is made of an elastic substance, and has a pair of arms 21a that can spread apart in a roughly V-shape toward the tip thereof, and claws 21b formed by bending the tips of the arms 21a inward. The base ends of the arms 21a are integrated into a roughly U-shape and connected to the distal end (tip) of the drive wire 23. The connecting hook 21 can be made of a metal such as stainless steel.

The connecting hook 21 protrudes from the distal end of the sheath 22 and opens its arms apart in a generally V-shape due to its own elasticity when the drive wire 23 is slid so as to push the drive wire 23 distally relative to the sheath 22 (i.e., the sheath 22 is slid proximally relative to the drive wire 23). Conversely, when the drive wire 23 is slid so as to retract the drive wire 23 proximally relative to the sheath 22 (i.e., the sheath 22 is slid distally relative to the drive wire 23), the connecting hook 21 is accommodated inside the sheath 22 and closes its arms.

In the suturing device 2 of this embodiment, the drive wire 23 is held at a fixed position, and the sheath 22 is pushed out or retracted, allowing the pair of arms 21a of the connecting hook 21 to be freely opened and closed (gripped or released) while keeping the connecting hook 21 at a fixed position.

The proximal end (base end) of the sheath 22 through which the drive wire 23 is inserted, is connected and fixed to the distal end (tip) of the base portion 27 through a luer locking mechanism having a base-side locking member 25 and a sheath-side locking member 26.

The slider portion 28 is slidably attached to the base portion 27. The proximal end of the drive wire 23 passes through the passage holes of the sheath-side locking member 26 and the base-side locking member 25 to reach the slider portion 28, and is detachably fixed to the slider portion 28 through a locking screw 29.

By sliding the slider portion 28 distally relative to the base portion 27, the connecting hook 21 attached to the distal end of the drive wire 23 is pushed out from the distal end of the sheath 22 and opens its arms apart in a roughly V-shape due to its own elasticity. Conversely, by sliding the slider portion 28 proximally relative to the base portion 27, the connecting hook 21 attached to the distal end of the drive wire 23 is recessed from the distal end of the sheath 22 and is housed inside the sheath 22.

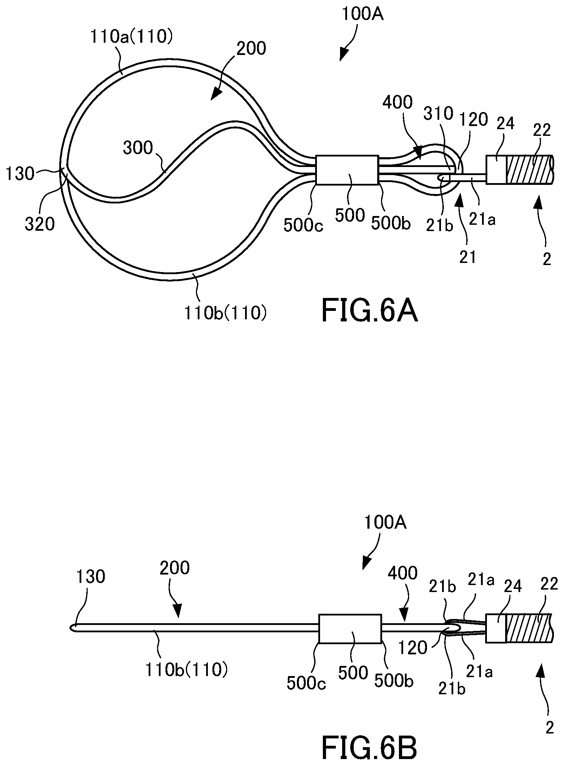

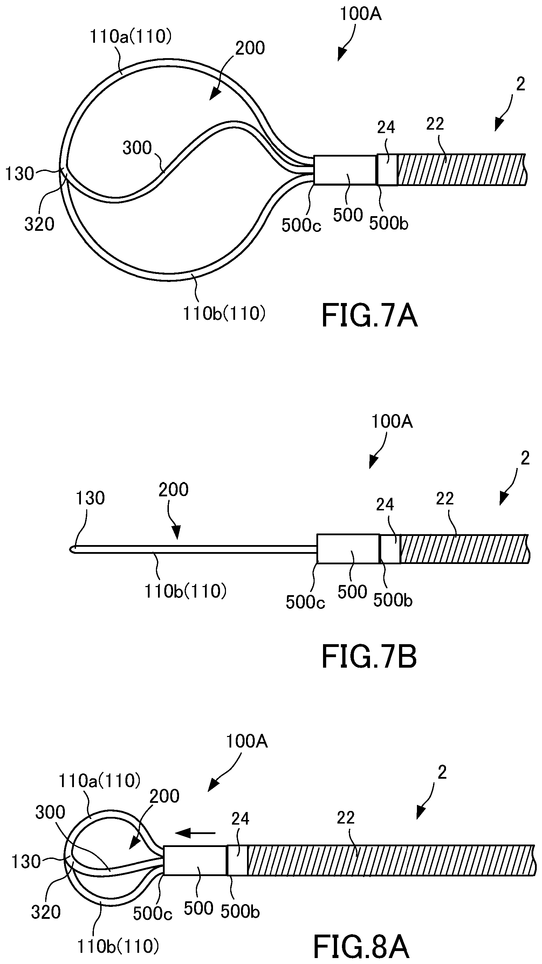

With reference to FIGS. 5A to 9, the operation of reducing the diameter of the loop portion 200 of the suturing member 100A using the above-described suturing device 2 will now be described. FIG. 5A is a plan view showing a state of the suturing member 100A in the first embodiment of the present invention immediately before being connected to the suturing device 2. FIG. 5B is a side view showing a state of the suturing member 100A in the first embodiment of the present invention immediately before being connected to the suturing device 2. FIG. 6A is a plan view showing a state in which the suturing member 100A in the first embodiment of the present invention is connected to the distal end of the suturing device 2. FIG. 6B is a side view showing a state in which the suturing member 100A in the first embodiment of the present invention is connected to the distal end of the suturing device 2. FIG. 7A is a plan view showing a state in which the distal end of the sheath 22 of the suturing device 2 abuts against the suturing member 100A in the first embodiment of the present invention. FIG. 7B is a side view showing a state in which the distal end of the sheath 22 of the suturing device 2 abuts against the suturing member 100A in the first embodiment of the present invention. FIG. 8A is a plan view showing a state in which the diameter of the suturing member 100A in the first embodiment of the present invention is contracted by the suturing device 2. FIG. 8B is a side view showing a state in which the diameter of the suturing member 100A in the first embodiment of the present invention is contracted by the suturing device 2. FIG. 9 is a plan view for explaining another example of the operation of contracting the diameter of the suturing member 100A in the first embodiment of the present invention by the suturing device 2.

When connecting the suturing member 100A to the distal end of the sheath 22 of the suturing device 2, the base portion 27 is slid proximally relative to the slider portion 28 to retract the sheath 22 relative to the drive wire 23. As shown in FIG. 5A and FIG. 5B, the connecting hook 21 at the distal end of the drive wire 23 protrudes from the distal end of the sheath 22 and opens the arms apart in a generally V-shape due to its own elasticity. The bridging wire 300 of the suturing member 100A is positioned so as to be contained within the loop surface LP of the loop portion 200. When viewed from the side as in FIG. 5B, for example, the bridging wire 300 is hidden behind the wire 110b located at the front of the drawing.

From this state, when the base portion 27 is slid distally relative to the slider portion 28, the sheath 22 is pushed out relative to the drive wire 23, and the connecting hook 21 at the distal end of the drive wire 23 enters the sheath 22, gradually closing the arms. The connecting hook 21 is positioned so that it can grasp the connecting loop portion 400 of the suturing member 100A, and the connecting hook 21 is housed within the sheath 22 to completely close the arms. This allows the connecting loop portion 400 of the suturing member 100A to be grasped by being pinched by the connecting hook 21, as shown in FIG. 6A and FIG. 6B.

When the base portion 27 is slid distally relative to the slider portion 28 while the connecting loop portion 400 of the suturing member 100A is gripped by the connecting hook 21, the sheath 22 is pushed out relative to the drive wire 23, and as shown in FIG. 7A and FIG. 7B, the distal end face of the tip member 24 provided at the distal end of the sheath 22 of the suturing device 2 abuts against the proximal end face (base end face) 500b of the tubular member 500 of the suturing member 100A.

From this state, the base portion 27 is slid further distally relative to the slider portion 28, and the sheath 22 is slid distally relative to the drive wire 23, thereby pushing out the tubular member 500 by the sheath 22. As a result, as shown in FIG. 8A and FIG. 8B, the tubular member 500 can be slid from the annular wire base end 120 toward the annular wire tip end 130, thereby squeezing the loop portion 200 to reduce its diameter.

To release the connection of the suturing member 100A to the distal end of the sheath 22, the base portion 27 is slid proximally relative to the slider portion 28, and the sheath 22 is slid proximally relative to the drive wire 23, causing the connecting hook 21 to protrude from the distal end of the sheath 22 and open. This releases the connection of the suturing member 100A to the connecting hook 21, allowing the suturing member 100A to be removed from the suturing device 2.

As shown in FIG. 9, while the wire portion in the vicinity of the proximal end face (base end face) 500b of the tubular member 500 of the suturing member 100A is laterally grasped by the connecting hook 21, the sheath 22 may be slid distally relative to the drive wire 23, sliding the tubular member 500 from the annular wire base end 120 toward the annular wire tip end 130. This action allows the tubular member 500 to be pushed out forcefully, tightly tightening the loop portion 200, and is particularly effective in the finishing stages of the suturing process.

With reference to FIG. 10A and FIG.10B, as well as FIG. 11A and FIG. 11B, a method for suturing a mucosal defect D using the suturing member 100A will be described. FIG. 10A is a diagram showing a state in which the suturing member 100A in the first embodiment of the present invention is secured near the mucosal defect D, as viewed from above the mucosal defect D. FIG. 10B is a diagram showing a state in which the suturing member 100A in the first embodiment of the present invention is secured near the mucosal defect D, as viewed from the side of the mucosal defect D. FIG. 11A is a diagram showing a state in which a mucosal defect D is sutured by contracting the diameter of the suturing member 100A in the first embodiment of the present invention, as viewed from above the mucosal defect D. FIG. 11B is a diagram showing a state in which a mucosal defect D is sutured by contracting the diameter of the suturing member 100A in the first embodiment of the present invention, as viewed from the side of the mucosal defect D. The suturing device 2 described above is not shown in FIG. 10A and FIG. 10B, as well as FIG. 11A and FIG. 11B. FIG. 10B and FIG. 11B illustrate a cross section of the mucosal defect D.

Below, as an example, a case will be described in which a suturing member 100A is used to suture a mucosal defect D that has occurred after a lesion in the gastrointestinal wall has been resected using a high-frequency scalpel or the like through ESD. When suturing the mucosal defect D, first, the suturing member 100A is attached to the distal end of the suturing device 2 and positioned near the mucosal defect D through a working channel of an endoscope not shown.

The endoscope used here may be a one-channel endoscope with a single working channel, or a multi-channel endoscope with two or more working channels. When using a one-channel endoscope, for example, the suturing member 100A is transported and positioned near the mucosal defect D using the suturing device 2, and then the device inserted into the working channel of the endoscope is replaced with a known clipping device or the like to secure the suturing member 100A with the clips.

After securement is complete, the device is replaced with the suturing device 2 and the suturing procedure can be performed. On the other hand, when using a multi-channel endoscope, the distal end of the suturing device 2 to which the suturing member 100A is attached is inserted through the first working channel, and a known clipping device or the like is inserted through the second working channel to secure the suturing member 100A with the clip. After this securement is complete, the suturing device 2 inserted through the first working channel can be operated to perform the suturing procedure.

Next, the suturing member 100A positioned near the mucosal defect D is secured using a plurality of endoscopic clips C (hereinafter referred to as clips C). Similarly to the clipping devices, known clips C can also be used.

In addition to the annular wire 110 that forms a substantially circular outline in a plan view, the loop portion 200 of the suturing member 100A also has a bridging wire 300 disposed therein to span across the inside of the loop portion 200. When using clips C to secure the wire, for example, the loop portion 200 is positioned so that the approximate center of the opening of the mucosal defect overlaps the approximate center of the loop portion 200, and the annular wire 110 is positioned so as to surround the opening of the mucosal defect. At this time, the bridging wire 300 inside the loop portion 200 is positioned so as to span across the opening of the mucosal defect.

In this state, the annular wire 110 is pressed against the peripheral mucosa while clamping with the clips C, thereby gripping (closing) the clips C. This allows the annular wire 110 to be secured to the peripheral mucosa by the clips C. The number and position of the clips C used to secure the annular wire 110 to the mucosa can be selected as appropriate.

The bridging wire 300, located inside the loop portion 200, is clamped by clips C and inserted into the mucosal defect D through the opening of the mucosal defect. The clips C are then pressed against the bottom surface Db (submucosa or muscular layer) of the mucosal defect to grasp (close) the bridging wire 300. This allows the clips C to secure the bridging wire 300 to the bottom surface Db of the mucosal defect. In particular, when the bridging wire 300 is curved in a roughly S-shape as shown in the figures, the bridging wire 300 can be made longer and have more slack, making it easier for the bridging wire 300 to reach the bottom surface Db of the mucosal defect, facilitating the task of securing the bridging wire 300 to the bottom surface Db of the mucosal defect. The number and positions of the clips C used to secure the bridging wire 300 to the bottom surface Db of the mucosal defect can be selected as appropriate.

Through the above procedure, as shown in FIG. 10A and FIG. 10B, the annular wire 110 constituting the loop portion 200 can be secured to the peripheral mucosa, and the bridging wire 300 constituting the loop portion 200 can be secured to the bottom surface Db of the mucosal defect. FIG. 10A and FIG. 10B show an example in which the annular wire 110 is secured to the peripheral mucosa using four clips C, and the bridging wire 300 is secured to the bottom surface Db of the mucosal defect using two clips C.

The clips C attached to the predetermined positions on the peripheral mucosa and the bottom surface Db of the mucosal defect grasp and secure the peripheral mucosa and the bottom surface Db of the mucosal defect. Meanwhile, the annular wire 110 and the bridging wire 300 grasped by the clips C are positioned so as to be prevented from disengaging from the secured positions of their respective clips C, but are still able to move (slide) along their longitudinal direction.

Next, the suturing member 100A secured to the mucosa near the mucosal defect D by the clip C is subjected to a suture procedure. Here, the loop portion 200 of the suturing member 100A is contracted, thereby suturing the mucosal defect D. Specifically, as described with reference to FIGS. 5A to 9, the tubular member 500 of the suturing member 100A is slid using the suturing device 2, thereby squeezing and contracting the loop portion 200.

As the tubular member 500 of the suturing member 100A is gradually slid from the annular wire base end 120 toward the annular wire annular wire tip end 130, the annular wire 110 and the bridging wire 300 of the loop portion 200 are drawn into the lumen 500a of the tubular member 500, gradually contracting the loop portion 200. At this time, in state that the clips C are secured to the peripheral mucosa or the bottom surface Db of the mucosal defect, the annular wire 110 and the bridging wire 300 move (slide) longitudinally, causing the loop portion 200 to contract in diameter. As a result, the peripheral mucosa or the bottom surface Db of the mucosal defect, where each clip C is secured, moves toward the approximate center of the loop portion 200. This allows the opening of the mucosal defect to be closed, as shown in FIG. 11A and FIG. 11B, and further allows the bottom surface Db of the mucosal defect to be lifted toward the opening of the mucosal defect.

In particular, the suturing member 100A in the first embodiment of the present invention is provided with the bridging wire 300 inside the loop portion 200. The bridging wire 300, positioned so as to hang over the mucosal defect D, can be inserted into the opening of the mucosal defect and the bottom surface Db of the mucosal defect can be secured with the clips C. Conventional purse-string suture techniques have the problem of forming a void (pocket) inside the mucosal defect D, as shown in FIG. 16B. On the other hand, when the mucosal defect D is sutured using the suturing member 100A in the first embodiment of the present invention, the bottom surface Db of the mucosal defect rises toward the opening of the mucosal defect, as shown in FIG. 11B. This reduces the void inside the mucosal defect D (or eliminates the void inside the mucosal defect D), allowing the bottom surface Db of the mucosal defect to be tightly adhered. This stops bleeding from the bottom surface Db of the mucosal defect and promotes healing of the mucosal defect D.

The tubular member 500 of the suturing member 100A is firmly pushed distally (toward the tip) and pressed in so as not to return to the proximal side (toward the base). The suturing member 100A is then detached from the suturing device 2, and the suturing device 2 of the suturing member 100A is removed from the body, completing the suturing procedure for the mucosal defect D. If necessary, portions of the annular wire 110 and the bridging wire 300 extending proximally beyond the tubular member 500 of the suturing member 100A may be cut and recovered using a high-frequency knife or similar device.

### (Second embodiment)

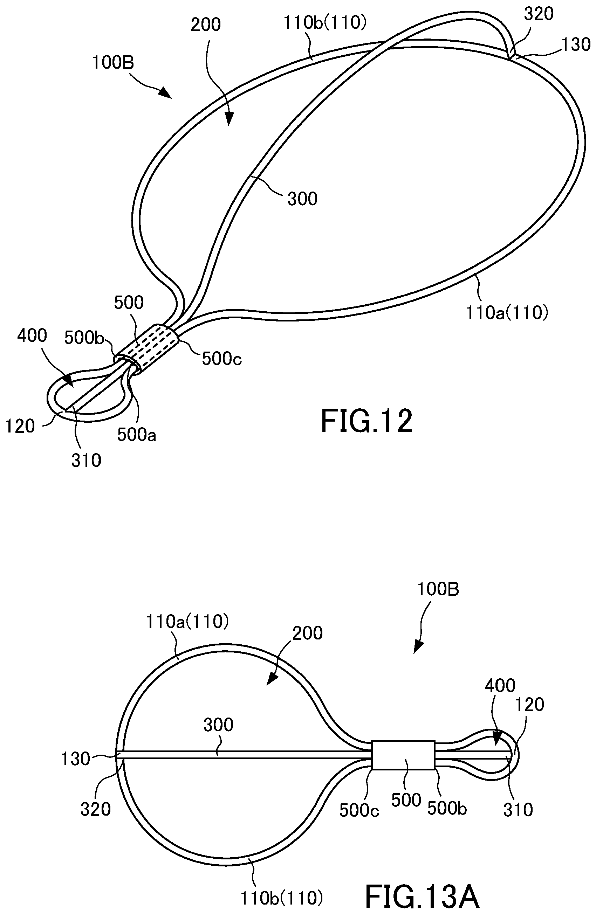

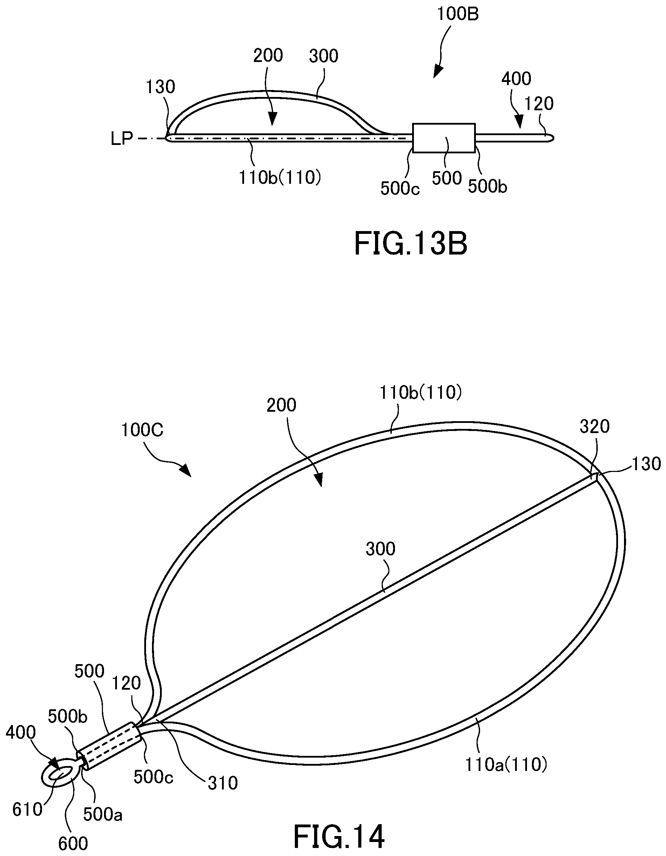

Next, the configuration of a suturing member 100B according to a second embodiment of the present invention will be described with reference to FIGS. 12, 13A, and 13B. FIG. 12 is a perspective view of a suturing member 100B in the second embodiment of the present invention. FIG. 13A is a plan view of the suturing member 100B in the second embodiment of the present invention. FIG. 13B is a side view showing the suturing member 100B in the second embodiment of the present invention. Hereinafter, the components similar to those of the suturing member 100A in the first embodiment described above are designated by the same reference numerals, and their description will be omitted or simplified.

As shown in FIGS. 12, 13A, and 13B, the suturing member 100B in the second embodiment, like the suturing member 100A in the first embodiment described above, has a configuration in which a tubular member 500 receives therein an annular wire 110 and a bridging wire 300. The loop portion 200 formed on the distal side (tip side) of the tubular member 500 has the annular wire 110 that forms a substantially circular outline in plan view and the bridging wire 300 that spans across the inside of the loop portion 200. Sliding the tubular member 500 from the annular wire base end 120 toward the annular wire tip end 130 allows the loop portion 200 to be squeezed and reduced in diameter.

In the suturing member 100A in the first embodiment described above, the bridging wire 300 is pre-shaped to be curved in a generally S-shape and is positioned so as to be contained within the loop surface LP of the loop portion 200. In contrast, in the suturing member 100B of the second embodiment, as shown in FIG. 13B, the bridging wire 300 is pre-shaped to be curved so as to protrude from the loop surface LP of the loop portion 200.

By thus curving the bridging wire 300 so as to protrude from the loop surface LP of the loop portion 200, the bridging wire 300 can be reliably lengthened. As a result, the bridging wire 300 can be given some slack, making it easier for the bridging wire 300 to reach the bottom surface Db of the mucosal defect, and thus facilitating securement of the bridging wire 300 to the bottom surface Db of the mucosal defect with the clips C. Furthermore, when the suturing member 100B is positioned near the mucosal defect D, the bridging wire 300 protrudes above the mucosal defect D, making it easy to secure the bridging wire 300 with the clips C. The length of the curved bridging wire 300 is not particularly limited, but, similar to the suturing member 100A in the first embodiment, the length of the curved bridging wire 300 is approximately the same as the length of the annular wire 110 from the annular wire base end 120 to the annular wire tip end 130.

### (Third embodiment)

Next, the configuration of the suturing member 100C in a third embodiment of the present invention will be described with reference to FIG. 14. FIG. 14 is a perspective view showing the suturing member 100C in the third embodiment of the present invention. Below, the components of the third embodiment similar to those in the suturing members 100A and 100B in the first and second embodiments described above will be designated by the same reference numerals, and their description will be omitted or simplified.

As shown in FIG. 14, the suturing member 100C of the third embodiment, like the suturing member 100A in the first embodiment described above, has a configuration in which a tubular member 500 receives therein an annular wire 110 and a bridging wire 300. The loop portion 200 formed on the distal side (tip side) of the tubular member 500 has an annular wire 110 that forms a substantially circular outline in plan view, and a bridging wire 300 that spans across the inside of the loop portion 200. Sliding the tubular member 500 from the annular wire base end 120 toward the annular wire tip end 130 allows the loop portion 200 to be squeezed and reduced in diameter.

In the first and second embodiments described above, the bridging wire 300 of the suturing members 100A and 100B is pre-shaped into a curved shape, whereas the bridging wire 300 of the suturing member 100C in the third embodiment is preliminarily formed into a generally straight shape, as shown in FIG. 14. However, as in the first and second embodiments described above, the bridging wire 300 may be pre-shaped into a generally S-shape or so as to protrude from the loop surface LP.

In the suturing member 100A in the first embodiment described above, the loop formed by the annular wire 110 extending from the vicinity of the proximal end face 500b of the tubular member 500 to the proximal side (base end side) of the tubular member 500 is used as the connecting loop portion 400 to be grasped by the connecting hook 21 of the suturing device 2. In contrast, as shown in FIG. 14, the suturing member 100C of the third embodiment has an annular connecting member 600 attached proximally (toward the base end) of the tubular member 500, which receives therein the annular wire 110 and the bridging wire 300. A through hole 610 is formed in the connecting member 600, and this through hole 610 can be used as a connecting loop portion 400 to be grasped by the connecting hook 21 of the suturing device 2.

By providing the annular connecting member 600 on the proximal side (base end) of the suturing member 100C in this way, the annular connecting member 600 can be securely grasped by the connecting hook 21 of the suturing device 2 when the tubular member 500 is slid from the annular wire base end 120 toward the annular wire tip end 130. The connecting member 600 may be made of a material (e.g., metal) different from that of the annular wire 110 and the bridging wire 300, and may be fixed by adhesive or other means to the proximal side (base end side) of the annular wire base end 120 where the annular wire 110 and the bridging wire 300 are connected.

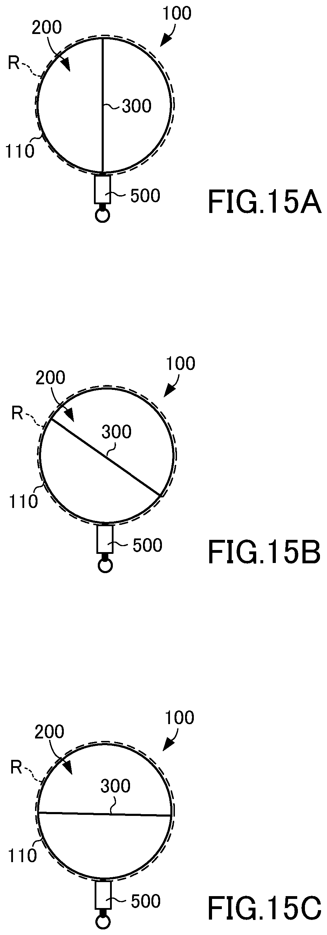

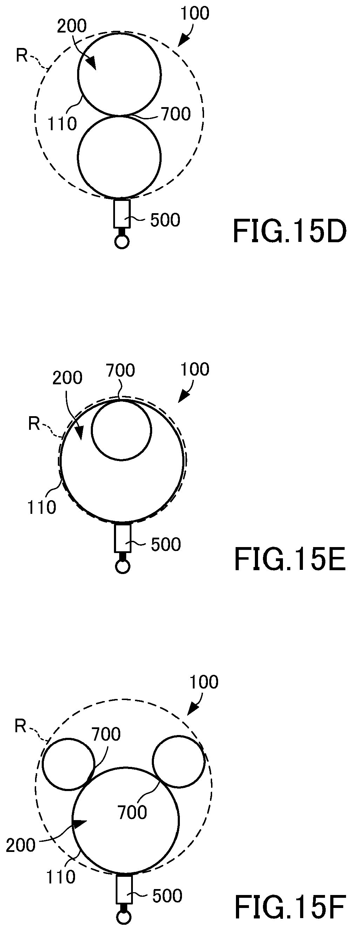

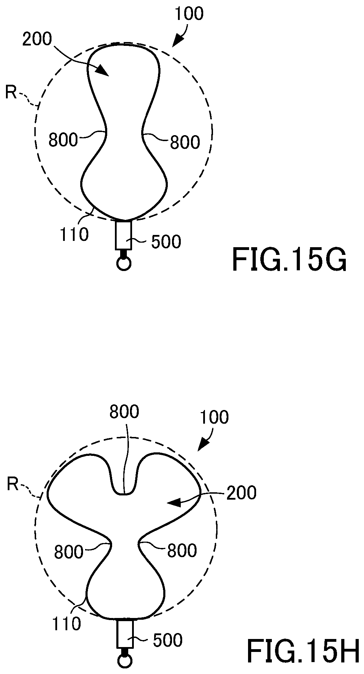

The concept of the present invention and the suturing member 100 included in the present invention will be described below. FIGS. 15A to 15H are schematic diagrams showing the shape of the loop portion 200 of the suturing member 100 according to the present invention. FIGS. 15A to 15C show first to third examples in which the bridging wire 300 spans across the inside of the loop portion 200. FIGS. 15D to 15F show first to third examples in which the wire constituting the loop portion 200 has an intersection portion 700. FIG. 15G and FIG. 15H show first and second examples in which the wire constituting the loop portion 200 has a concavely curved portion 800.

Detachable snares conventionally used in purse-string suturing (e.g., detachable snare 900 shown in FIG. 16A and FIG. 16B) have a loop portion on the distal side (tip side), similar to the suturing members 100A, 100B, and 100C in the first to third embodiments described above. However, the loop portion formed on conventional detachable snares is generally circular, and the wire is not positioned inside the reference circle that characterizes the outer shape of the loop portion (the reference circle that encompasses the loop portion). The reference circle that encompasses the loop portion here refers to a circle that characterizes the outer shape (contour) of the loop portion, and is the smallest encompassing circle or smallest circumscribing circle of the loop portion.

On the other hand, the suturing member 100 according to the present invention is configured to have a wire that is positioned inside the reference circle that characterizes the outer shape of the loop portion 200 (the reference circle that encompasses the loop portion 200). With this configuration, when the suturing member 100 is positioned so that the approximate center of the loop portion 200 (the center of the reference circle) overlaps the approximate center of the mucosal defect D, the wire can be positioned above the mucosal defect D, and the annular wire 110 forming the loop portion 200 can be secured to the peripheral mucosa with the clips, while the wire positioned above the mucosal defect D can be inserted into the mucosal defect D and secured to the bottom surface Db of the mucosal defect with the clips. The present invention encompasses suturing members 100 of any shape configured so that the wire is positioned inside the reference circle that characterizes the outer shape of the loop portion 200 (the reference circle that encompasses the loop portion 200).

The suturing member 100 according to the present invention may have a substantially straight or curved bridging wire 300 disposed to span across the inside of the loop portion 200. For example, in each of the suturing members 100 shown in FIGS. 15A to 15C, a bridging wire 300 is provided to connect two different points on the annular wire 110 that forms the loop portion 200, and the bridging wire 300 is positioned inside a reference circle R that encompasses the loop portion 200. When suturing a mucosal defect D, the bridging wire 300 is positioned above the mucosal defect D.

The suturing member 100 shown in FIG. 15A is provided with a bridging wire 300 that coincides with the sliding direction of the tubular member 500. Representative examples of the suturing member 100 shown in FIG. 15A are the suturing members 100A, 100B, and 100C of the first to third embodiments described above. Furthermore, the suturing members 100 shown in FIG. 15B and FIG. 15C are provided with a bridging wire 300 that extends diagonally or perpendicularly to the sliding direction of the tubular member 500. Furthermore, multiple bridging wires 300 may be provided.

Furthermore, the suturing member 100 according to the present invention may include the annular wire 110 constituting the loop portion 200, the annular wire 110 being pre-shaped to have an intersection portion 700 at which the annular wire 110 intersects with itself. At the intersection portion 700, the annular wire 110 constituting the loop portion 200 is in the overlapped state or in the twisted state. For example, in all of the suturing members 100 shown in FIGS. 15D to 15F, the annular wire 110 constituting the loop portion 200 has an intersection portion 700, and as the wire overlaps or twists at the intersection portion 700, the wire is positioned inside a reference circle R that encompasses the loop portion 200. When suturing a mucosal defect D, a portion of the wire constituting the annular wire 110 is positioned above the mucosal defect D. The position and number of intersection portions 700 are not particularly limited.

Furthermore, the suturing member 100 according to the present invention may include the annular wire 110 constituting the loop portion 200, the annular wire 110 being pre-shaped to have a concavely curved portion 800 that is curved so as to be recessed inward of the loop portion 200. For example, the suturing members 100 shown in FIG. 15G and FIG. 15H both have a concavely curved portion 800 that is recessed inward of the loop portion 200. Due to the inward recess caused by the concavely curved portion 800, the wire is positioned inside a reference circle R that encompasses the loop portion 200. When suturing a mucosal defect D, a portion of the wire constituting the annular wire 110 is positioned above the mucosal defect D. The position and number of concavely curved portions 800 are not particularly limited.

The effects of the suturing member 100 according to the present invention will now be described.

The suturing member 100 according to the present invention is used to suture a mucosal defect D in a digestive tract wall. Like the suturing members 100A, 100B, and 100C in the first to third embodiments described above, the suturing member 100 comprises an annular wire 110 that branches into two wire portions at an annular wire base end 120 and is connected at an annular wire tip end 130 opposing to the annular wire base end 120 to form an annular shape, and a tubular member 500 that is slidable from the annular wire base end 120 toward the annular wire tip end 130. The annular wire 110 forms a loop portion 200 at a distal side of the tubular member 500. Furthermore, when the approximate center of the loop portion 200 of the suturing member 100 is positioned so as to overlap the approximate center of the mucosal defect D, the wire of the suturing member 100 is positioned above the mucosal defect D, and the tubular member 500 receives therein the annular wire 110 and the wire positioned above the mucosal defect D.

With the above configuration, when suturing the mucosal defect D, the wire of the suturing member 100 is positioned above the mucosal defect D. Therefore, the portion of the annular wire 110 that is positioned above the peripheral mucosa can be secured to the peripheral mucosa with an endoscopic clip (clip C) or the like, and the portion of the wire that is positioned above the mucosal defect D can be inserted into the mucosal defect D and secured to the bottom surface Db of the mucosal defect D (submucosa or muscularis) which is the bottom of the mucosal defect D, with the clips C or the like.

With this securement in place, when the tubular member 500 is slid toward the annular wire tip end 130, the loop portion 200 is squeezed and reduced in diameter, and the positions secured by the clip C or the like converge to approximately the center of the loop portion 200. This makes it possible to close the opening of the mucosal defect D and to lift the bottom surface Db of the mucosal defect D toward the opening the mucosal defect D, thereby reducing the space inside the mucosal defect D. This makes it possible to stop bleeding from the bottom surface Db of the mucosal defect D and promote healing of the mucosal defect D.

The suturing member 100 according to the present invention may include a bridging wire 300 connecting two different points on the annular wire 110 in the loop portion 200, as in the suturing members 100A, 100B, and 100C of the first to third embodiments described above, as a wire positioned above the mucosal defect D.

With the above configuration, the bridging wire 300 spanning across the inside of the loop portion 200 can be positioned above the mucosal defect D.

The suturing member 100 according to the present invention may have one end 310 of the bridging wire 300 connected to the annular wire base end 120 and the other end 320 of the bridging wire 300 connected to the annular wire tip end 130, as in suturing members 100A, 100B, and 100C of the first to third embodiments described above.

With the above configuration, the bridging wire 300 is disposed so that the extension direction of the bridging wire 300 coincides with the sliding direction of the tubular member 500. This allows the tubular member 500 to slide smoothly toward the annular wire tip end 130 after being secured with the clips C or the like.

The suturing member 100 according to the present invention may have a curved bridging wire 300, as in suturing members 100A and 100B of the first and second embodiments described above.

With the above configuration, the bridging wire 300 can be lengthened to provide sufficient slack, preventing deformation of the loop portion 200 due to the tension of the bridging wire 300 and allowing the loop surface LP of the loop portion 200 to be a flat surface that can be easily positioned near the mucosal defect D. Furthermore, lengthening the bridging wire 300 makes it easier to insert the bridging wire 300 into the mucosal defect D and to secure the bridging wire 300 to the bottom surface Db of the mucosal defect with the clips C or the like.

In the suturing member 100 according to the present invention, as in the suturing members 100A and 100B of the first and second embodiments described above, the length of the bridging wire 300 may be approximately the same as the length of the annular wire 110 from the annular wire base end 120 to the annular wire tip end 130.

With the above configuration, when the annular wire base end 120 and the annular wire tip end 130 are pulled in opposite directions to form a shape that brings them closer to each other, the bridging wire 300 becomes in a state of not being loosed. Therefore, when the tubular member 500 is slid toward the annular wire tip end 130 after being secured with the clips C or the like, the annular wire 110 and the bridging wire 300 is prevented from being slack, thereby allowing the tubular member 500 to slide smoothly toward the annular wire tip end 130.

The suturing member 100 according to the present invention may have the bridging wire 300 included within the loop surface LP of the loop portion 200 and curved in a substantially S-shape, as in the suturing member 100A in the first embodiment described above.

With the above configuration, the bridging wire 300 is reliably lengthened to provide sufficient slack, making it easier for the bridging wire 300 to reach the bottom surface Db of the mucosal defect. This makes it easy to secure the bridging wire 300 to the bottom surface Db of the mucosal defect with the clips C or the like.

The suturing member 100 according to the present invention may be configured such that the bridging wire 300 may be curved so as to protrude from the loop surface LP of the loop portion 200, as in the suturing member 100B of the second embodiment described above.

With the above configuration, the bridging wire 300 is reliably lengthened to provide sufficient slack, making it easier for the bridging wire 300 to reach the bottom surface Db of the mucosal defect. This makes it easy to secure the bridging wire 300 to the bottom surface Db of the mucosal defect with the clips C or the like. Furthermore, when the suturing member 100B is positioned near the mucosal defect D, the bridging wire 300 is disposed to protrude above the mucosal defect D, making it easy to secure the bridging wire 300 with the clips C or the like.

In the suturing member 100 according to the present invention, as shown in FIGS. 15D to 15F, for example, the wires constituting the annular wire 110 are pre-shaped to have, in the loop portion 200, an intersection portion 700 at which the wires intersect with each other, and the wire positioned above the mucosal defect D may be formed from a portion of the wire constituting the annular wire 110.

With this configuration, when the approximate center of the loop portion 200 is positioned so as to overlap the approximate center of the mucosal defect D, a portion of the annular wire 110 having the intersection portion 700 can be positioned above the mucosal defect D.

In the suturing member 100 according to the present invention, as shown in FIG. 15G and FIG. 15H, for example, the annular wire 110 may be pre-shaped to have a concavely curved portion 800 that is curved so as to be recessed inward of the loop portion 200, and the wire positioned above the mucosal defect D may be formed from a portion of the wire constituting the annular wire 110.

With the above configuration, when the approximate center of the loop portion 200 is positioned so as to overlap the approximate center of the mucosal defect D, the concavely curved portion 800, which is a portion of the annular wire 110, can be positioned above the mucosal defect D.

The suturing member 100 according to the present invention may also include a connecting loop portion 400 formed in an annular shape at a position closer to the proximal end than the tubular member 500, as in the suturing members 100A, 100B, and 100C of the first to third embodiments described above.

With the above configuration, when the tubular member 500 is slid toward the annular wire tip end 130 after being secured with the clips C or the like, the annular connecting loop portion 400 can be grasped by the suturing device 2, allowing the tubular member 500 to slide smoothly.

In the suturing member 100 according to the present invention, the connecting loop portion 400 may be formed from the annular wire 110, similarly to the suturing members 100A and 100B of the first and second embodiments described above.

With the above configuration, the connecting loop portion 400 can be formed from a portion of the annular wire 110, allowing the connecting loop portion 400 to be provided with a simple configuration.

In the suturing member 100 according to the present invention, the connecting loop portion 400 may be formed from an annular connecting member 600 attached to the annular wire 110, similarly to the suturing member 100C of the third embodiment described above.

With the above configuration, the connecting loop portion 400 can be formed using the annular connecting member 600 that can be gripped during suturing, without being limited by the structure of the annular wire 110.

The above-described embodiments have been described to facilitate understanding of the present invention and are not intended to limit the present invention. The components disclosed in the above-described embodiments are intended to include all design modifications and equivalents that fall within the technical scope of the present invention. Furthermore, configurations obtained by appropriately combining the components described in each embodiment are also included in the present invention.

### EXPLANATION OF REFERENCE NUMERALS AND SIGNS

- 2: Suturing device
- 2A: Locking mechanism
- 2B: Operating unit
- 21: Connecting hook
- 21a: Arm
- 21b: Claw
- 22: Sheath
- 23: Drive wire
- 24: Tip member
- 25: Base-side locking member
- 26: Sheath-side locking member
- 27: Base portion
- 28: Slider portion
- 29: Locking screw
- 100, 100A, 100B, 100C: Suturing member
- 110: Annular wire
- 110a, 110b: Wire
- 120: Annular wire base end
- 130: Annular wire tip end
- 200: Loop portion
- 300: Bridging wire
- 310: One end
- 320: Other end
- 400: Connecting loop portion
- 500: Tubular member
- 500a: Lumen
- 500b: Proximal end face (base end face)
- 500c: Distal end face (tip end face)
- 600: Connecting member
- 610: Through hole
- 700: Intersection portion
- 800: Concavely curved portion
- 900: Detachable snare
- 910: Tubular member
- C: Clip (endoscopic clip)
- D: Mucosal defect
- Db: Bottom surface of mucosal defect
- LP: Loop surface
- R: Reference circle

## Claims

1. A suturing member used to suture a mucosal defect in a digestive tract wall, comprising:
an annular wire branching into two wire portions at an annular wire base end and connected at an annular wire tip end opposing the annular wire base end to form an annular shape; and
a tubular member slidable from the annular wire base end toward the annular wire tip end, the annular wire forming a loop portion at a distal side of the tubular member,
the suturing member further comprising a wire positioned above the mucosal defect when the approximate center of the loop portion overlaps the approximate center of the mucosal defect,
the tubular member receiving therein the annular wire and the wire positioned above the mucosal defect.

2. The suturing member according to claim 1, wherein the wire positioned above the mucosal defect is constituted by a bridging wire formed to connect two different points of the annular wire in the loop portion.

3. The suturing member according to claim 2, wherein one end of the bridging wire is connected to the annular wire base end and the other end of the bridging wire is connected to the annular wire tip end.

4. The suturing member according to claim 2, wherein the bridging wire is curved.

5. The suturing member according to claim 4, wherein the length of the bridging wire is approximately the same as the length of the annular wire from the annular wire base end to the annular wire tip end.

6. The suturing member according to claim 4, wherein the bridging wire is included within the loop surface of the loop portion and is curved in a substantially S-shape.

7. The suturing member according to claim 4, wherein the bridging wire is curved so as to protrude from the loop surface of the loop portion.

8. The suturing member according to claim 1, wherein the wires constituting the annular wire are pre-shaped to have, in the loop portion, an intersection portion at which the wires intersect with each other, and the wire positioned above the mucosal defect is formed from a portion of the wire constituting the annular wire.

9. The suturing member according to claim 1, wherein the annular wire is pre-shaped to have a concavely curved portion that is curved so as to be recessed inward of the loop portion, and the wire positioned above the mucosal defect is formed from a portion of the wire constituting the annular wire.

10. The suturing member according to any one of claims 1 to 9, comprising a connecting loop portion formed in an annular shape at a position closer to the proximal end than the tubular member.

11. The suturing member according to claim 10, wherein the connecting loop portion is formed from the annular wire.

12. The suturing member according to claim 10, wherein the connecting loop portion is formed from an annular connecting member attached to the annular wire.
